(19) Europäisches Patentamt
European Patent Office
Office européen des brevets

(11) Veröffentlichungsnummer: **0 010 727 B1**

(12) **EUROPÄISCHE PATENTSCHRIFT**

(45) Veröffentlichungstag der Patentschrift: **18.01.84**

(21) Anmeldenummer: **79104113.0**

(22) Anmeldetag: **23.10.79**

(51) Int. Cl.³: **C 07 C 69/747,**
C 07 C 33/26,
C 07 C 43/29,
C 07 C 67/14, A 01 N 53/00

(54) **Vinylcyclopropancarbonsäure-3-phenoxy-alpha-vinylbenzylester, Verfahren zu ihrer Herstellung und ihre Verwendung als Schädlingsbekämpfungsmittel.**

(30) Priorität: **26.10.78 CH 11077/78**
**11.09.79 CH 8204/79**

(43) Veröffentlichungstag der Anmeldung:
**14.05.80 Patentblatt 80/10**

(45) Bekanntmachung des Hinweises auf die Patenterteilung:
**18.01.84 Patentblatt 84/3**

(84) Benannte Vertragsstaaten:
**AT BE CH DE FR GB IT NL**

(56) Entgegenhaltungen:
**EP - A - 0 001 824**
**US - A - 3 973 036**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Patentabteilung Postfach**
**CH-4002 Basel (CH)**

(72) Erfinder: **Drabek, Jozef, Dr.**
**Benkenstrasse 12**
**CH-4104 Oberwil (CH)**
Erfinder: **Ackermann, Peter, Dr.**
**Reichensteinerstrasse 12**
**CH-4153 Reinach (CH)**
Erfinder: **Farooq, Saleem, Dr.**
**Im Schaiengarten 2**
**CH-4107 Ettingen (CH)**
Erfinder: **Gsell, Laurenz, Dr.**
**Maiengasse 56**
**CH-4056 Basel (CH)**
Erfinder: **Kristiansen, Odd, Dr.**
**Delligrabenstrasse 7**
**CH-4313 Möhlin (CH)**

(74) Vertreter: **Zumstein, Fritz sen., Dr. et al,**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Vinylcyclopropancarbonsäure-3-phenoxy-alpha-vinylbenzylester, Verfahren zu ihrer Herstellung und ihre Verwendung als Schädlingsbekämpfungsmittel

Die vorliegende Erfindung betrifft Vinylcyclopropancarbonsäure-3-phenoxy-$\alpha$-vinylbenzylester, Verfahren zu ihrer Herstellung und ihre Verwendung in der Schädlingsbekämpfung.

Die Vinylcyclopropancarbonsäure-3-phenoxy-$\alpha$-vinylbenzylester haben die Formel

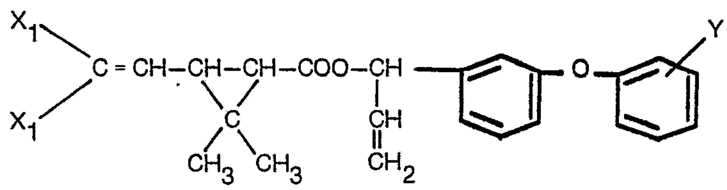

(I) -

worin $X_1$ Fluor, Chlor oder Brom, und
Y Fluor oder Brom bedeuten.

Die Verbindungen der Formel I werden nach an sich bekannten Methoden z.B. wie folgt hergestellt:

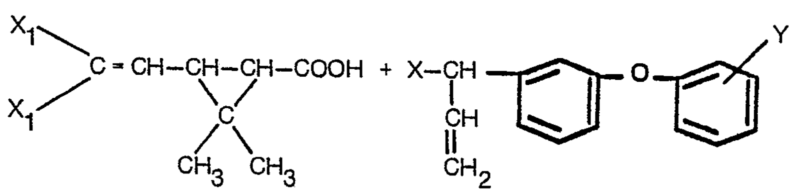

(II) (III)

2)

säurebindendes

Mittel

I

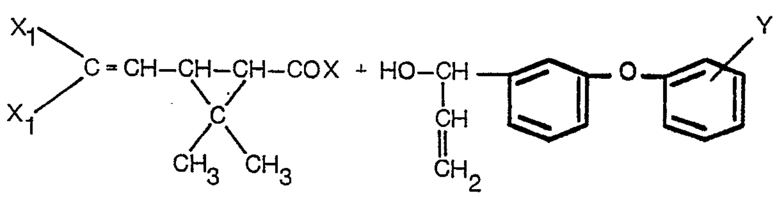

(IV) (V)

saurebindendes

Mittel

I

3)

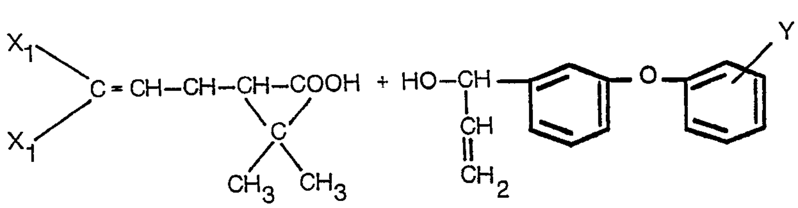

(II) (V)

wasserbindendes

Mittel

I

4)

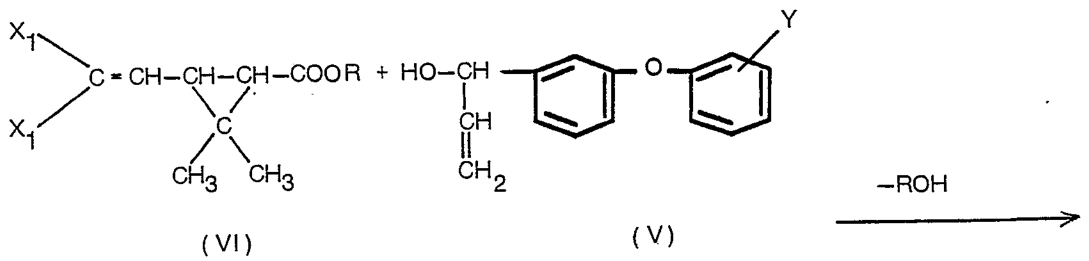


(VI) (V) I

In den Formeln II bis VI haben $X_1$ und Y die für die Formel I angegebene Bedeutung.

In den Formeln III und IV steht X für ein Halogenatom, insbesondere Chlor oder Brom und in der Formel VI steht R für $C_1$—$C_4$-Alkyl, inbesondere für Methyl oder Aethyl. Als säurebindendes Mittel für die Verfahren 1 und 2 kommen insbesondere tertiäre Amine, wie Trialkylamin und Pyridin, ferner Hydroxide, Oxide, Carbonate und Bicarbonate von Alkali- und Erdalkalimetallen sowie Alkalimetallalkoholate wie z.B. Kalium-t.butylat und Natriummethylat in Betracht. Als wasserbindendes Mittel für das Verfahren 3 kann z.B. Dicyclohexylcarbodiimid verwendet werden. Die Verfahren 1 bis 4 werden bei einer Reaktionstemperatur zwischen −10 und 120°C, meist zwischen 20 und 80°C bei normalem oder erhöhtem Druck und vorzugsweise in einem inerten Lösungs- oder Verdünnungsmittel durchgeführt. Als Lösungs- oder Verdünnungsmittel eignen sich z.B. Aether und ätherartige Verbindungen wie Diäthyläther, Dipropyläther, Dioxan, Dimethoxyäthan und Tetrahydrofuran; Amide wie N,N-dialkylierte Carbonsäureamide, aliphatische, aromatische sowie halogenierte Kohlenwasserstoffe, insbesondere Benzol, Toluol, Xylol, Chloroform and Chlorbenzol; Nitrile wie Acetonitril; Dimethylsulfoxid und Ketone wie Aceton und Methyläthylketon.

Die Ausgangsstoffe der Formeln II, IV und VI sind bekannt und können analog bekannten Methoden hergestellt werden. Die Verbindungen der Formel V sind neu. Sie werden aus den Alkoholen der Formel

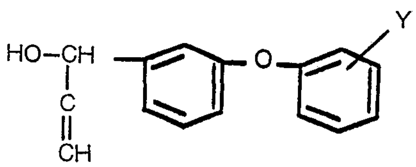

mittels Reduktion oder aus den Aldehyden der Formel

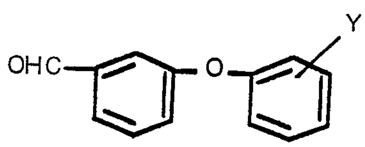

durch Grignard-Reaktion mit X-Mg-CH=$CH_2$ (X gleich Chlor oder Brom) hergestellt.

Die Verbindungen der Formel I liegen als Gemisch von verschiedenen optisch aktiven Isomeren vor, wenn bei der Herstellung nicht einheitlich optisch aktive Ausgangsmaterialien verwendet wurden. Die verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von vershiedenartigen tierischen und pflanzlichen Schädlingen.

Insbesondere eignen sich die Verbindungen der Formel I zur Bekämpfung von Insekten, phytopathogenen Milben und von Zecken z.B. der Ordnungen Lepidoptera, Coleoptera, Homptera, Heteroptera, Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z.B. gegen Spodoptera littoralis und Heliothis virescens) und Gemüsekulturen (z.B. gegen Leptinotarsa decemlineata und Myzus persicae). Aus der U.S. Patentschrift Nr. 3,973,036 sind Vinylcyclopropancarbonsäure-3-phenoxy-$\alpha$-äthinylbenzylester bekannt, die akarizide und insektizide wirkung aufweisen.

Die Verbindungen der Formel I haben auch eine sehr gute Wirkung gegen keratinfressende Lepidoptera, z.B. Tineola spec. und Tinea spec., sowie gegen keratinfressende Coleoptera, z.B. Anthrenus spec. und Attagenus spec. Die Verbindungen eignen sich deshalb sehr gut zum Schützen von keratinischen Materialien gegen Befall durch Schädlinge. Die Verbindungen der Formel I lassen sich nach den für die Textilausrüstung üblichen Verfahren anwenden und eignen sich daher vorzüglich zum Schützen von textilem Material gegen Insektenfrass, insbesondere zur wasch- und lichtechten Ausrüstung von derartigen Materialien, so wohl in rohem als auch in verarbeitetem Zustand; z.B. von roher oder verarbeiteter Schafwolle, sowie anderen Tierhaaren, Fellen, Pelzen und Federn.

Von den zu schützenden Materialien sind auch solche zu erwähnen, deren eine Komponente aus Wolle besteht während die andere eine andere Naturfaser, wie z.B. Baumwolle, oder eine Kunstfaser ist. Neben der licht- und waschechten Ausrüstung im Färbebad und in der Foulardapplikation können die Verbindungen der Formel I aber auch zur Imprägnierung von Wolle und wollenen Artikeln bei der Trockenreinigung dienen, wodurch ein ebenfalls vorzüglicher Frass-Schutz erzielt wird.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxa- undecan (Sesamex resp. Sesoxana), S,S,S-Tributylphosphorotrithioate, 1,2-Methylendioxy-4-(2-(octylsulfonyl)-propyl)-benzol, N-(2-Ethylhexyl)-bicyclo-(2,2,1)-heptone(2)-2,3-dicarboxamid.

Verbindungen der Formel I können für sich oder zusammen mit geeigneten Trägern und/oder Zuschlagstoffen eingesetzt werden. Geeignete Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln.

Die Herstellung erfindungsgemässer Mittel erfolgt in an sich bekannter Weise durch inniges Vermischen und/oder Vermahlen der Wirkstoffe der Formel I mit den geeigneten Trägerstoffen, gegebenenfalls unter Zusatz von gegenüber den Wirkstoffen inerten Dispergier- oder Lösungsmitteln. Die Wirkstoffe können in den folgenden Aufarbeitungsformen vorliegen und angewendet werden.:

Feste Aufarbeitungsformen: Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate: Spritzpulver (wettable powders), Pasten, Emulsionen;

b) Lösungen

Der Gehalt an Wirkstoff in den oben beschriebenen Mitteln liegt zwischen 0,1 bis 950, dabei ist zu erwähnen, dass bei der Applikation aus dem Flugzeug oder mittels anderer geeigneter Applikationsgeräte Konzentrationen bis zu 99,5% oder sogar reiner Wirkstoff eingesetzt werden können. Die Wirkstoffe der Formel I können beispielsweise wie folgt formuliert werden (Teile bedeuten Gewichtsteile):

*Stäubemittel:* Zur Herstellung eines a) 5%igen und b) 2%igen Stäubemittels werden die folgenden Stoffe verwendet:

a) 5 Teile Wirkstoff
95 Teile Talkum;

b) 2 Teile Wirkstoff
1 Teil hochdisperse Kieselsäure
97 Teile Talkum.

Der Wirkstoff wird mit den Trägerstoffen vermischt und vermahlen.

*Granulat*: Zur Herstellung eines 5%igen Granulates werden die folgenden Stoffe verwendet:

5 Teile Wirkstoff
0,25 Teile epoxydiertes Pflanzenöl
0,25 Teile Cetylpolyglykoläther
3,50 Teile Polyäthylenglykol
91 Teile Kaolin (Korngrösse 0,3—0,8 mm);

Die Aktivsubstanz wird mit dem epoxydierten Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

*Spritzpulver:* Zur Herstellung eines a) 40%igen, b) und c) 25%igen, d) 10%igen Spritzpulvers werden folgende Bestandteile verwendet:

a) 40 Teile Wirkstoff
5 Teile Ligninsulfonsäure-Natriumsalz
1 Teil Dibutylnaphthalinsulfonsäure-Natriumsalz
54 Teile Kieselsäure;

b) 25 Teile Wirkstoff
4,5 Teile Calcium-Ligninsulfonat
1,9 Teile Champagne-Kreide/Hydroxyäthylcellulose-Gemisch (1:1)
1,5 Teile Natrium-dibutyl-naphthalinsulfonat
19,5 Teile Kieselsäure
19,5 Teile Champagne-Kreide
28,1 Teile Kaolin;

c) 25 Teile Wirkstoff
2,5 Teile Isooctylphenoxy-polyäthylen-äthanol
1,7 Teile Champagne-Kreide/Hydroxyäthylcellulose-Gemisch (1:1)
8,3 Teile Natriumaluminiumsilikat
16,5 Teile Kieselgur
46 Teile Kaolin;

d) 10 Teile Wirkstoff
3 Teile Gemisch der Natriumsalze von gesättigten Fettalkoholsulfaten
5 Teile Naphthalinsulfonsäure/Formaldehyd-Kondensat
82 Teile Kaolin.

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

*Emulgierbare Konzentrate:* Zur Herstellung eines a) 10%igen b) 25%igen und c) 50%igen emulgierbaren Konzentrates werden folgende Stoffe verwendet.

a) 10 Teile Wirkstoff
3,4 Teile epoxydiertes Pflanzenöl
3,4 Teile eines Kombinationsemulgators, bestehend aus Fettalkoholpolyglykoläther und Alkylarylsulfonat-Calcium-Salz
40 Teile Dimethylformamid
43,2 Teile Xylol;

b) 25 Teile Wirkstoff
2,5 Teile epoxydiertes Pflanzenöl
10 Teile eines Alkylarylsulfonat/Fettalkoholpolyglykoläther-Gemisches
5 Teile Dimethylformamid
57,5 Teile Xylol;

c) 50 Teile Wirkstoff
4,2 Teile Tributylphenol-Polyglykoläther
5,8 Teile Calcium-Dodecylbenzolsulfonat
20 Teile Cyclohexanon
20 Teile Xylol.

Aus solchen Konzentraten können durch Verdünnen mit Wasser Emulsionen jeder gewünschten Konzentration hergestellt werden.

*Sprühmittel:* Zur Herstellung eines a) 5%igen und b) 95%igen Sprühmittels werden die folgenden Bestandteile verwendet:

a) 5 Teile Wirkstoff
1 Teil epoxydiertes Pflanzenöl
94 Teile Benzin (Siedegrenzen 160—190°C);

b) 95 Teile Wirkstoff
5 Teile epoxydiertes Pflanzenöl

Beispiel 1

a) Herstellung von 3-(4-Fluorphenoxy)-$\alpha$-vinylbenzylalkohol

145,3 g 3-(4-Fluorphenoxy)-$\alpha$-äthinylbenzylalkohol werden in 1,5 l Dioxan gelöst. In diese Lösung werden 30 g Lindlar Katalysator zugegeben. Unter Schütteln werden in das Gemisch 14,15 l (105% der Theorie) Wasserstoff während 45 Minuten eingeleitet. Danach wird die Apparatur mit

Stickstoff gespült, das Reaktionsgemisch abgenutscht und das Lösungsmittel abdestilliert.

Man erhält den Alkohol der Formel

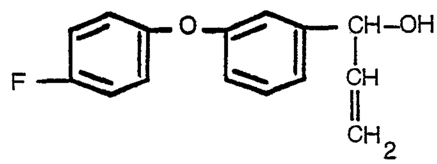

als Oel mit einer Refraktion von $n_D^{23°} = 1{,}5690$.

Auf analoge Weise werden auch folgende Alkohole hergestellt:

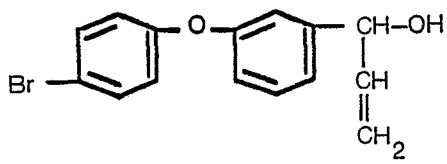

$n_D^{23,5°} = 1{,}6087$

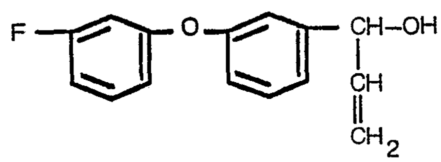

$n_D^{21°} = 1{,}5710$

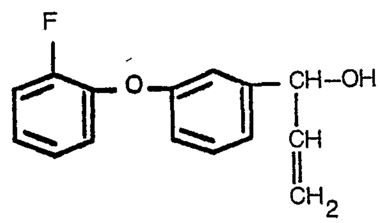

$n_D^{21°} = 1{,}5709$

b) Herstellung von 2,2-Dimethyl-3-(2,2-dichlorvinyl)-cyclopropancarbonsäure-3-(4-fluorphenoxy)-$\alpha$-vinyl-benzyl-ester

Man tropft bei 5°C 2,4 ml Pyridin in 5 ml Toluol zu einer Lösung von 4,9 g 3-(4-Fluorphenoxy)-$\alpha$-vinylbenzylalkohol in 20 ml Toluol. Bei 10°C werden dann 4,55 g 2,2-Dimethyl-3-(dichlor-vinyl)-cyclopropan-carbonsäurechlorid (80% ± cis, 20% ± trans) zugetropft. Man rührt anschliessend 2 Stunden bei Raumtemperatur und lässt 12 Stunden stehen. Zur Aufarbeitung verdünnt man das Reaktionsgemisch mit Eis-wasser, extrahiert die organische Phase 3 mal mit je 100 ml 3%iger Salzsäurelösung und 3 mal mit je 100 ml 3%iger Natriumbicarbonatlösung, trocknet über Natriumsulfat und destilliert das Toluol ab.

Man erhält die Verbindung der Formel

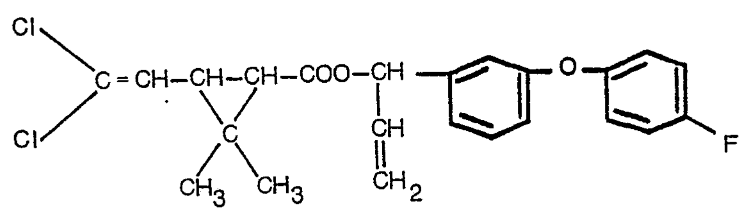

als farbloses Oel mit einer Refraktion von $n_D^{40°} = 1{,}5488$. Auf analoge Weise werden auch folgende Verbindungen hergestellt:

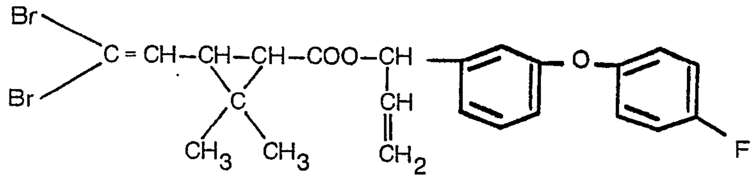

$n_D^{40°} = 1,5665$

(ca. 60 % ± trans; 40 % ± cis)

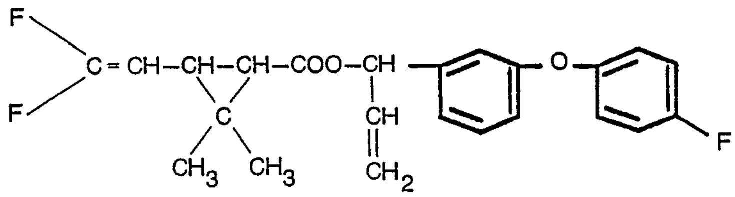

$n_D^{40°} = 1,5179$

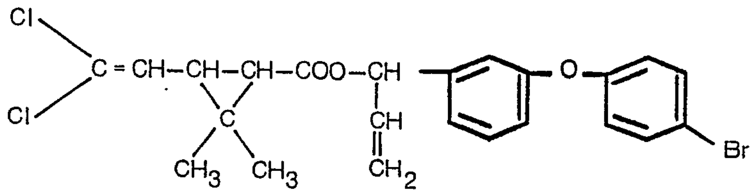

$n_D^{40°} = 1,5190$

(ca. 80 % ± cis; 20 % ± trans)

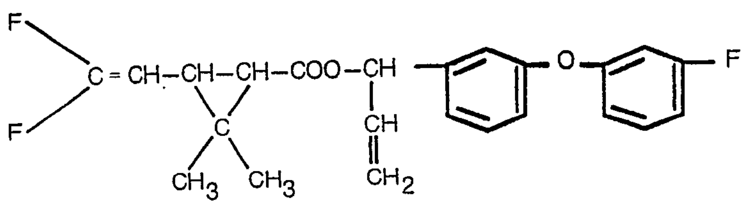

$n_D^{40°} = 1,5193$

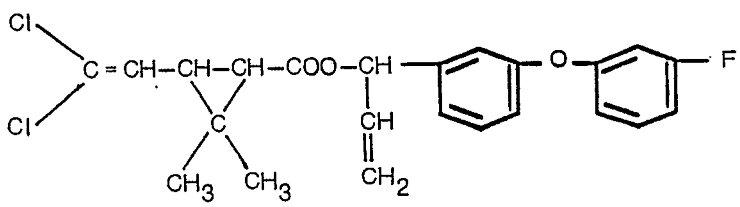

$n_D^{40°} = 1,5487$

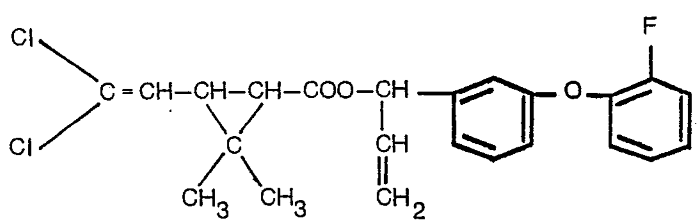

$n_D^{40°} = 1,5499$

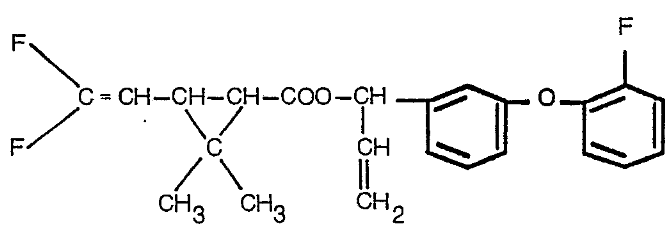

Beispiel 2

A) Insektizide Frassgift-Wirkung

Baumwollpflanzen wurden mit einer 0,05 %igen wässrigen Wirkstoffemulsion (erhalten aus einem 10 %igen emulgierbaren Konzentrat) besprüht.

Nach dem Antrocknen des Belages wurden die Baumwollpflanzen je mit Spodoptera littoralis- und Heliothis virescens-Larven $L_3$ besetzt. Der Versuch wurde bei 24°C und 60% relativer Luftfeuchtigkeit durchgeführt.

Verbindungen gemäss Beispiel 1 (b), zeigten im obigen Test eine gute insektizide Frassgift-Wirkung gegen Spodoptera- und Heliothis-Larven.

Beispiel 3

Akarizide Wirkung

Phaseolus vulgaris Pflanzen wurden 12 Stunden vor dem Test auf akarizide Wirkung mit einem infestierten Blattstück aus einer Massenzucht von Tetraanychus urticae belegt. Die übergelaufenen beweglichen Stadien wurden aus einem Chromatographiezerstäuber mit den emulgierten Testpräparaten derart besprüht, dass kein Ablaufen der Spritzbrühe eintrat. Nach zwei und 7 Tagen wurden Larven, Adulte und Eier unter dem Binokular auf lebende und tote Individuen ausgewartet und das Ergebnis in Prozenten ausgedrückt. Während der "Haltezeit" standen die behandelten Pflanzen in Gewächshauskabinen bei 25°C.

Verbindungen gemäss Beispiel 1 (b) wirkten im obigen Test gegen Adulte, Larven und Eier von Tetranychus urticae.

Beispiel 4

*Wirkung gegen Zecken*

A) Rhipicephalus bursa

Je 5 adulte Zecken bzw. 50 Zeckenlarven wurden in ein Glasröhrchen gezählt und für 1 bis 2 Minuten in 2 ml einer wässrigen Emulsion aus einer Verdünnungsreihe mit je 100, 10, 1 oder 0,1 ppm Testsubstanz getaucht. Das Röhrchen wurde dann mit einem genormten Wattebausch verschlossen und auf den Kopf gestellt, damit die Wirkstoffemulsion von der Watte aufgenommen werden konnte.

Die Auswertung erfolgte bei den Adulten nach 2 Wochen und bei den Larven nach 2 Tagen. Für jeden Versuch liefen 2 Wiederholungen.

B) Boophilus microplus (Larven)

Mit einer analogen Verdünnungsreihe wie beim Test A) wurden mit je 20 sensiblen resp. OPresistenten Larven Versuche durchgeführt. (Die Resistenz bezieht sich auf die Verträglichkeit von Diazinon). Verbindungen gemäss Beispiel 1 (b) wirkten in diesen Tests gegen Adulte und Larven von Rhipicephalus bursa und sensible resp. OP-resistente Larven von Boophilus microplus.

**Patentansprüche für die Vertragsstaaten: BE CH DE FR GB IT NL**

1. Eine Verbindung der Formel

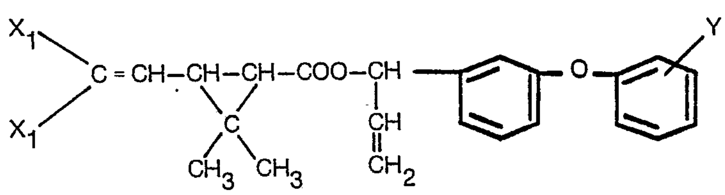

worin $X_1$ Fluor, Chlor oder Brom und Y Fluor oder Brom bedeuten.

2. Die Verbindung gemäss Anspruch 1 der Formel

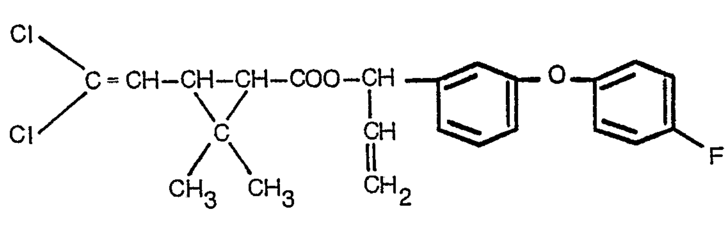

3. Die Verbindung gemäss Anspruch 1 der Formel

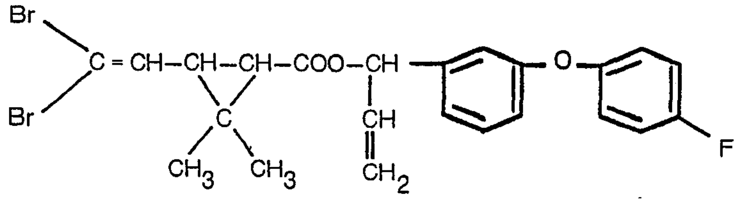

4. Die Verbindung gemäss Anspruch 1 der Formel

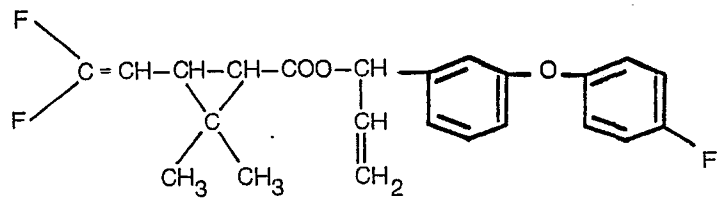

5. Die Verbindung gemäss Anspruch 1 der Formel

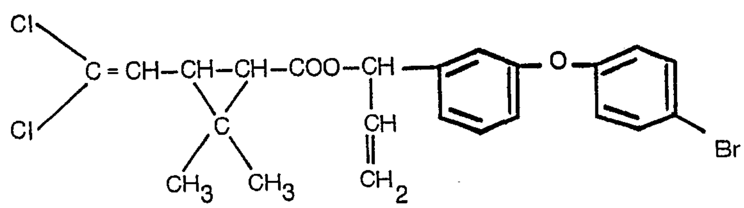

6. Verfahren zur Herstellung einer Verbindung gemäss Patentanspruch 1, dadurch gekennzeichnet, dass man eine Verbindung der Formel

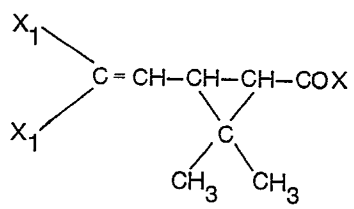

in Gegenwart eines säurebindenden Mittels mit einer Verbindung der Formel

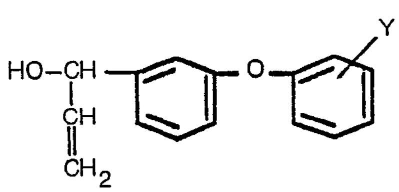

umsetzt, worin $X_1$ und Y die im Anspruch 1 angegebene Bedeutung haben und X für ein Halogenatom steht.

7. Ein Schädlingsbekämpfungsmittel, welches als aktive Komponente eine Verbindung gemäss Anspruch 1 und geeignete Träger- und/oder andere Zuschalgstoffe enthält.

8. Verwendung einer Verbindung gemäss Anspruch 1 zur Bekämpfung von verschiedenartigen tierischen und pflanzlichen Schädlingen.

9. Verwendung gemäss Anspruch 8 zur Bekämpfung von Insekten und Vertretern der Ordnung Akarina.

10. Eine Verbindung der Formel

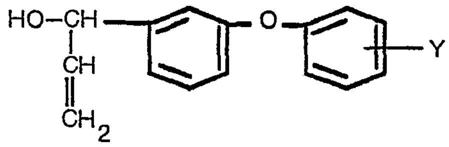

worin Y Fluor oder Brom bedeutet.

**Patentansprüche für den Vertragsstaat: AT**

1. Schädlingsbekämpfungsmittel, enthaltend als aktive Komponente eine Verbindung der Formel

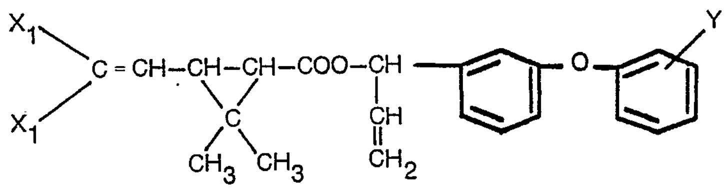

worin $X_1$ Fluor, Chlor oder Brom und Y Fluor oder Brom bedeuten, und geeignete Träger- und/oder andere Zuschlagstoffe.

2. Schädlingsbekämpfungsmittel nach Anspruch 1, enthaltend als aktive Komponente eine Verbindung der Formel

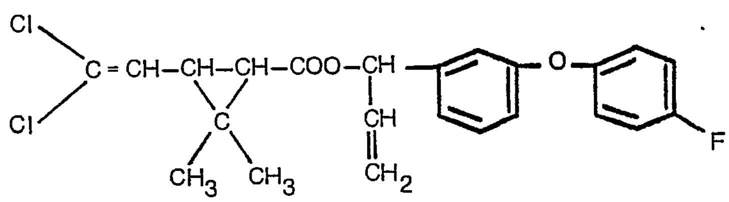

3. Schädlingsbekämpfungsmittel nach Anspruch 1, enthaltend als aktive Komponente eine Verbindung der Formel

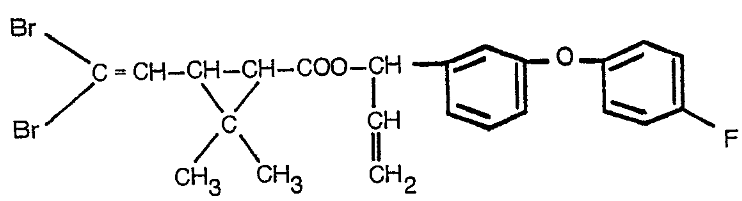

4. Schädlingsbekämpfungsmittel nach Anspruch 1, enthaltend als aktive Komponente eine Verbindung der Formel

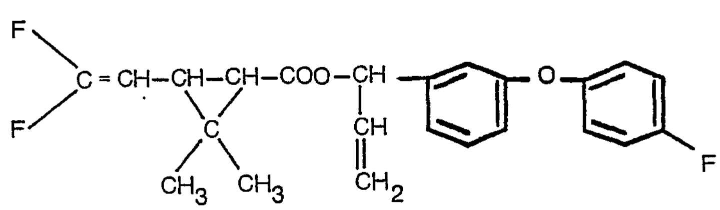

5. Schädlingsbekämpfungsmittel nach Anspruch 1, enthaltend als aktive Komponente eine Verbindung der Formel

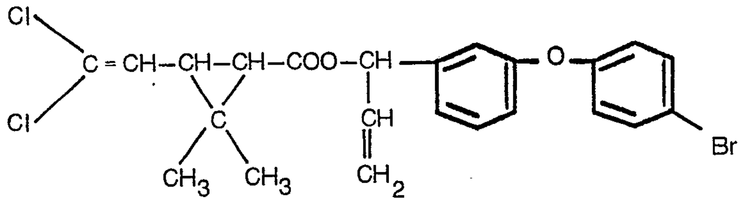

6. Schädlingsbekämpfungsmittel nach Anspruch 1 zur Bekämpfung von verschliedenartigen tierischen und pflanzlichen Schädlingen.

7. Schädlingsbekämpfungsmittel nach Anspruch 6 zur Bekämpfung von Insekten und Vertretern der Ordnung Akarina.

**Revendications pour les Etats contractants: BE CH DE FR GB IT NL**

1. Composé de formule

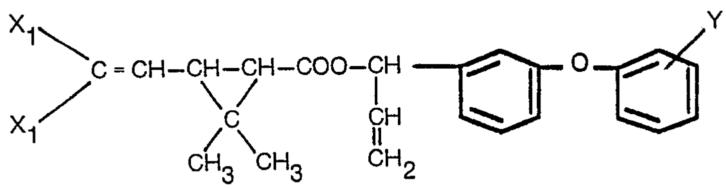

où $X_1$ représente un fluor, un chlore ou un brome et Y représente un fluor ou un brome.

2. Composé selon la revendication 1 de formule

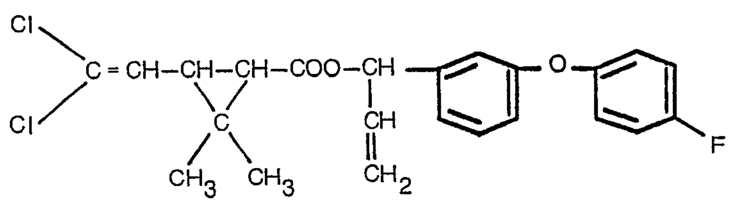

3. Composé selon la revendication 1 de formule

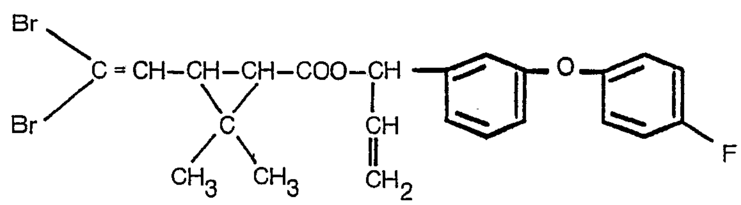

4. Composé selon la revendication 1 de formule

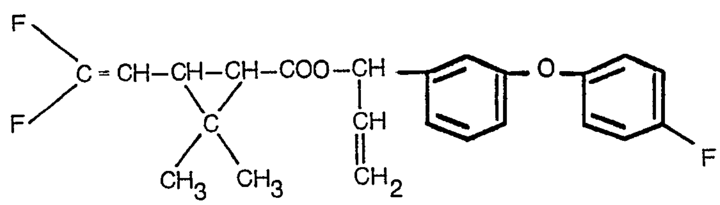

5. Composé selon la revendication 1 de formule

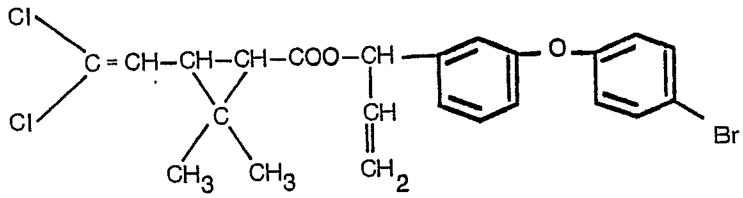

6. Procédé de préparation d'un composé selon la revendication 1, caractérisé en ce qu'on fait réagir un composé de formule

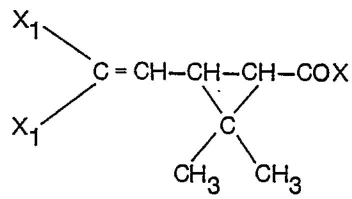

en présence d'un agent liant acide avec un composé de formule

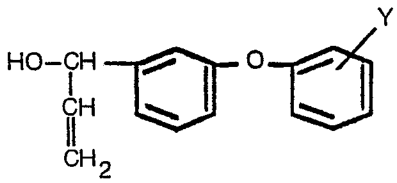

où $X_1$ et Y ont la signification donnée ci-dessus et X représente un atome d'halogène.

7. Agent de lutte antiparasitaire contenant comme composant actif un composé selon la revendication 1 et des supports et/ou autres additifs appropriés.

8. Application d'un composé selon la revendication 1 à la lutte contre différentes espèces de parasites animaux et végétaux.

9. Application selon la revendication 8 à la luttre contre les insectes et les représentants de l'ordre des acariens.

10. Composé de formule

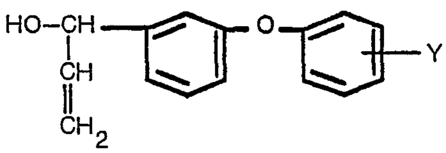

où Y représente un fluor ou un brome.

**Revendications pour l'Etat contractant: AT**

1. Agent de lutte antiparasitaire contenant comme composant actif un composé de formule

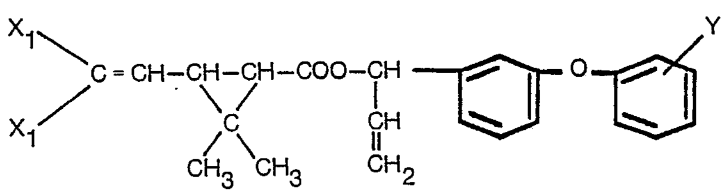

où $X_1$ représente un fluor, un chlore ou un brome et Y représente un fluor ou un brome, et des supports et/ou autres additifs appropriés.

2. Agent de lutte antiparasitaire selon la revendication 1, contenant comme composant actif un composé de formule

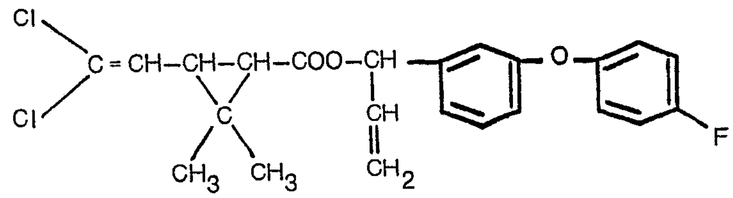

3. Agent de lutte antiparasitaire selon la revendication 1, contenant comme composant actif un composé de formule

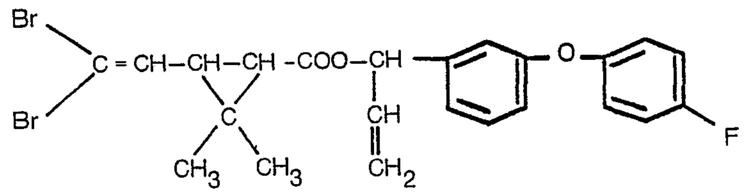

4. Agent de lutte antiparasitaire selon la revendication 1 contenant comme composant actif un composé de formule

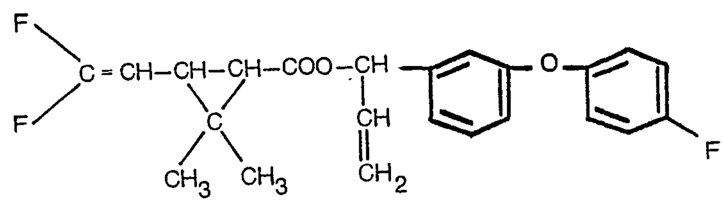

5. Agent de lutte antiparasitaire selon la revendication 1, contenant comme composant actif un composé de formule

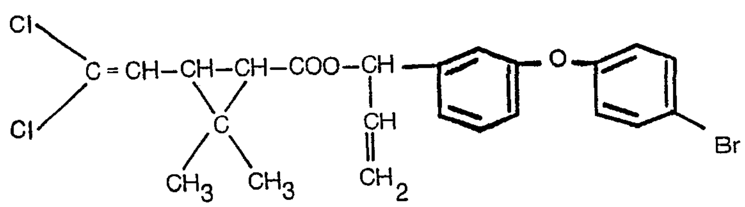

6. Agent de lutte antiparasitaire selon la revendication 1 pour lutter contre différentes espèces de parasites animaux et végétaux.

7. Agent de lutte antiparasitaire selon la revendication 6 pour lutter contre les insectes et les représentants de l'ordre des acariens.

**Claims for the Contracting States: BE CH DE FR GB IT NL**

1. A compound of formula

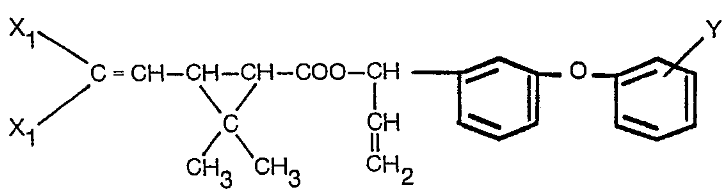

wherein $X_1$ is fluorine, chlorine or bromine and Y is fluorine or bromine.

2. The compound according to claim 1 of the formula

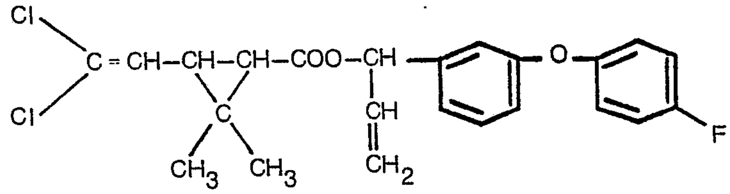

3. The compound according to claim 1 of the formula

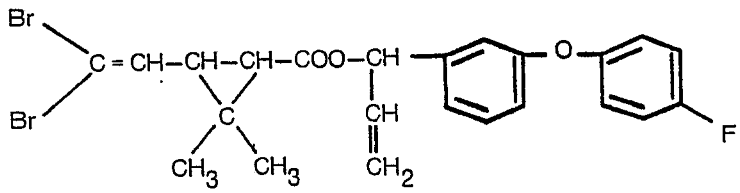

4. The compound according to claim 1 of the formula

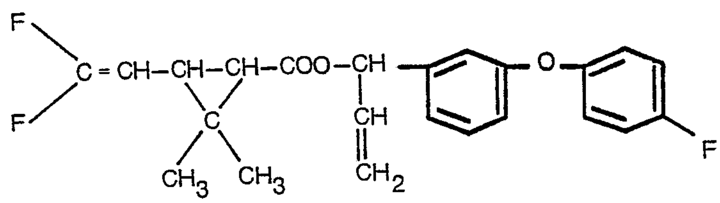

5. The compound according to claim 1 of the formula

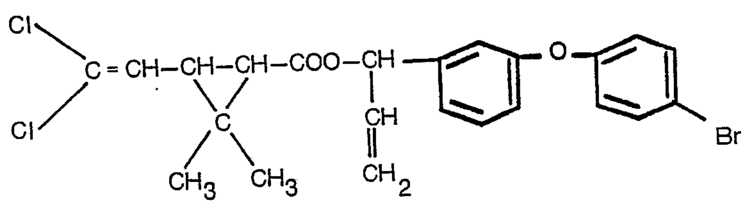

6. A process for the manufacture of a compound according to claim 1, which comprises reacting a compound of the formula

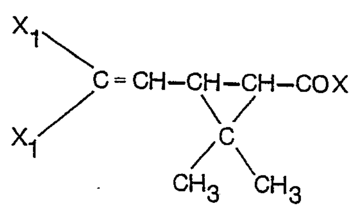

wherein $X_1$ is as defined in claim 1 and X is a halogen atom, with a compound of the formula

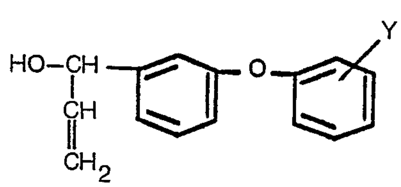

wherein Y is as defined in claim 1, in the presence of an acid acceptor.

7. A pesticidal composition which contains, as active component, a compound according to claim 1, together with suitable carriers and/or adjuvants.

8. A method of controlling a variety of animal and plant pests, which comprises the use of a compound according to claim 1.

9. A method according to claim 8, wherein the pests to be controlled are insects and representatives of the order Acarina.

10. A compound of the formula

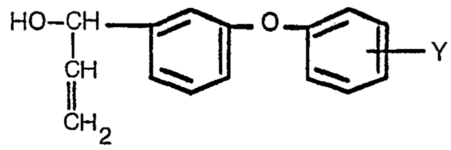

wherein Y is fluorine or bromine.

**Claims for the Contracting State: AT**

1. A pesticidal composition containing as active component a compound of the formula:

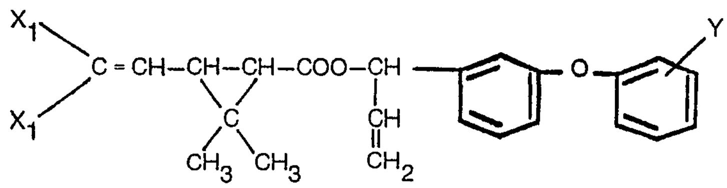

wherein $X_1$ is fluorine, chlorine or bromine and Y is fluorine or bromine, together with suitable carriers and/or other adjuvants.

2. A pesticidal composition according to claim 1, containing as active component a compound of the formula

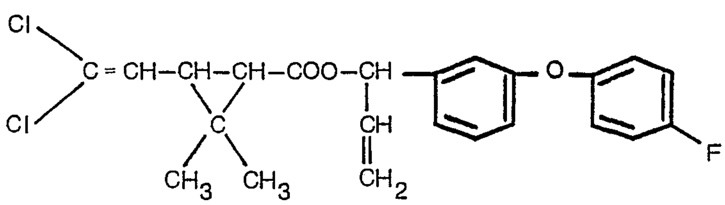

3. A pesticidal composition according to claim 1, containing as active component a compound of the formula

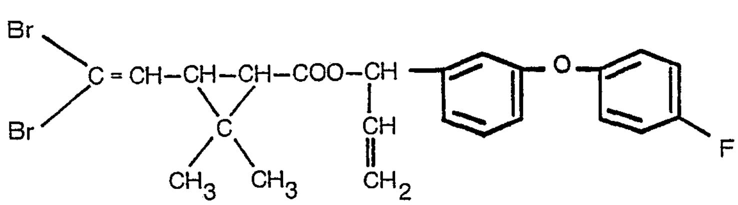

4. A pesticidal composition according to claim 1, containing as active component a compound of the formula

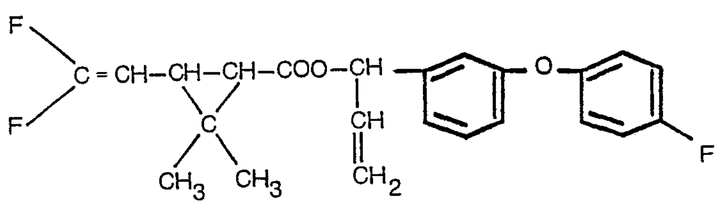

5. The compound according to claim 1 of the formula

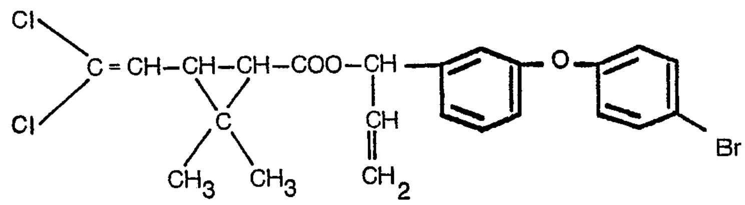

6. A pesticidal composition according to claim 1 for controlling a variety of animal and plant pests.

7. A pesticidal composition according to claim 6 for controlling insects and representatives of the order Acarina.